# EUROPÄISCHE PATENTSCHRIFT

(11) **EP 0 582 896 B1**
(45) Veröffentlichungstag und Bekanntmachung des Hinweises auf die Patenterteilung: **11.06.1997**
(21) Anmeldenummer: 93112085.1
(22) Anmeldetag: 28.07.1993
(51) Int. Cl.: A61C 1/00, A61C 1/08, A61C 17/00, A61C 17/02

(54) **Vorrichtung zur Bearbeitung von Zahnhartsubstanz**
Device for tooth treatment
Dispositif pour le traitement dentaire

(30) Priorität: 11.08.1992 DE 4226612
(43) Veröffentlichungstag der Anmeldung: 16.02.1994
(73) Patentinhaber: SIEMENS AKTIENGESELLSCHAFT, 80333 München (DE)
(72) Erfinder: Franetzki, Manfred, Dr., D-64625 Bensheim (DE)

(56) Entgegenhaltungen:
- EP-A- 0 280 823
- DE-A- 3 439 584
- US-A- 465 265
- US-A- 4 858 001
- US-A- 4 865 021
- US-A- 4 917 603

## Beschreibung

Die Erfindung bezieht sich auf eine Vorrichtung zur Bearbeitung von Zahnhartsubstanz, mit einem in den Patientenmund einführbaren Bearbeitungshandstück, mit dem ein Kühlmedium wie Luft und/oder Wasser der Präparationsstelle zugeführt werden kann, in Verbindung mit einer Absaugeinrichtung und einer intraoralen Videokamera, deren Zielobjekt auf einem Monitor wiedergegeben wird.

Solche Bearbeitungshandstücke können luft-, elektromotorisch- oder auch ultraschallbetriebene Handstücke oder auch Laserhandstücke sein, mit denen sich unterschiedliche Präparationen am Zahn durchführen lassen, z.B. Aufbereiten einer Kavität, Beschleifen eines Zahnes oder Entfernen von Zahnstein. Bei all diesen Präparationsarbeiten wird in der Regel der Präparationsstelle ein Kühlmittel, z.B. Kühlwasser oder Spray zugeführt.

Hinsichtlich der Ausgestaltung und Anordnung der intraoralen Videokamera, die sowohl an sich (EP-A-0 280 823, US-A-4 858 001) als auch in Verbindung mit einem Bearbeitungshandstück mit rotierendem Werkzeug (US-A-5 049 070) sowie in Verbindung mit einem Bearbeitungshandstück, welches Laserstrahlung abgibt (DE-U 92 04 412) bekannt ist, sind mehrere Varianten denkbar. So kann die Videokamera, zumindest die zur Bilderfassung notwendigen Teile, und eine geeignete Beleuchtungseinrichtung, im Munde, z. B. an einem gegenüberliegenden Zahn, fixiert sein, sie kann mit dem Saughandstück gekoppelt sein, oder sie kann im Bearbeitungshandstück integriert angeordnet sein, wie dies aus den vorgenannten beiden Dokumenten DE-U-9 204 412 und US-A-5 049 070 bekannt ist. Gegebenenfalls können in einem solchen Bearbeitungshandstück auch eine Kamera und eine Saugkanüle integriert angeordnet sein.

Nachteilig bei allen Varianten ist, daß die zugeführten Kühlmittel während der Bearbeitung eine Aerosolwolke bilden, mit der auch Keime mitgerissen werden, die dann, trotz der üblichen Absaugung, in das weitere Umfeld der zahnärztlichen Praxis getragen werden.

Der im Anspruch 1 angegebenen Erfindung liegt die Aufgabe zugrunde, eine Vorrichtung anzugeben, mit der vermieden werden kann, daß die im Patientenmund bei einer Präparation gebildete Aerosolwolke und die in ihr enthaltenen Teilchen und Partikel unkontrolliert aus dem Patientenmund austreten können, ohne daß dabei die Sicht auf die Präparationsstelle beeinträchtigt wird.

Die Erfindung fußt auf der Erkenntnis, durch Zusammenwirken einer indirekten Präparationstechnik mit einer am Patientenmund anzubringenden Abschirmung unter Zuhilfenahme einer Absaugeinrichtung außerhalb des Patientenmundes ein weitgehend keimfreies oder zumindest keimarmes Umfeld, soweit es eine vom Patientenmund ausgehende Keimkontaminierung anbelangt, zu schaffen. Die erfindungsgemäß vorgeschlagene Vorrichtung erlaubt somit ein Arbeiten mit indirekter Sicht unter hygienisch besonders günstigen Bedingungen.

Besondere Vorzüge ergeben sich bei einem in jüngster Zeit wieder aktuell gewordenen Bearbeitungsverfahren, bei dem anstelle eines rotierenden oder oszillierenden Bearbeitungswerkzeuges ein mit abrasiven Teilchen versetzter Luftstrom mit hoher Geschwindigkeit auf die Präparationsstelle gelenkt wird.

Die Abtragrate wird dabei bestimmt durch die kinetische Energie der Teilchen, der Menge pro Zeiteinheit (Massestrom), der Härte und der Schlagzähigkeit der Teilchen. Die kinetische Energie wird bestimmt durch die Dichte, die Größe und die Geschwindigkeit der Teilchen.

Durch geeignete Wahl dieser Parameter läßt sich die Wirkung auf die zu bearbeitende Substanz optimieren, etwa Grobbearbeiten von Zahnschmelz, Feinabtrag von Dentin in Pulpanähe oder Entfernen von Zahnbelag.

Da im Mund verbleibende Partikel stören können, ist die Verwendung von sich auflösenden Abrasionsmitteln, z.B. Salze, vorteilhaft.

Mit den erfindungsgemäß vorgeschlagenen Maßnahmen läßt sich nicht nur die Aerosolwolke mit eventuell darin enthaltenen Keimen, sondern auch, wenn das zuletzt angesprochene Verfahren angewendet wird, das bei einer Präparation anfallende Abrasionsmaterial abfangen und in geeigneter Weise entsorgen.

Die Abschirmung kann ein vor dem Patientenmund angeordnetes und vorteilhafterweise am Patientenkopf befestigtes Tuch sein, unter dem hindurch die Hände mit dem Bearbeitungshandstück in den Patientenmund greifen. Anstelle eines Tuches kann auch eine geeignete Folie vorgesehen werden. Die vom Tuch bzw. von der Folie abgehaltene Aerosolwolke kann nicht nach außen dringen; sie wird vielmehr während der Präparation in der üblichen Form über entsprechende Absaugmittel aus dem Patientenmund abgesaugt. Bei Anwendung des vorgenannten Salz- bzw. Sandstrahlverfahrens fällt das während der Präparation anfallende Material auf einen auf Hals und Brust des Patienten liegenden Latz, mit dem es nach der Behandlung leicht entsorgt werden kann. Mit der in den Mund eingeführten Absaugkanüle läßt sich die Staub- bzw. Spraywolke entfernen, wobei gleichzeitig ein leichter Unterdruck im Patientenmund erzeugt wird.

Alternativ kann die Abschirmung auch aus einem Schlauch aus Gewebe oder weichem Papier bestehen, wobei die eine Schlauchöffnung direkt an den Patientenmund angelegt (geheftet, gebunden, geklemmt) wird; durch die andere Öffnung können die Instrumente eingeführt werden. Zweckmäßigerweise ist zumindest dasjenige Ende, an dem die Instrumente eingeführt werden, besonders elastisch ausgebildet, so daß sich der Schlauch um die Hand und die Instrumente des Behandlers gut anschmiegt.

Eine einfache Alternative sieht vor, vor den Patientenmund eine elastische "Binde" zu spannen, die den Patientenmund dicht abschließt (ähnlich einem Kofferdamm). Durch vorgestanzte Schlitze können die Bearbeitungswerkzeuge eingeschoben und es kann damit behandelt werden.

Die optischen Fenster der Videokamera und der Beleuchtungseinrichtung sind vorteilhafterweise mittels eines transparenten Überzugs, der zweckmäßigerweise als Einmalartikel ausgebildet und leicht zu wechseln ist, gegen Beschädigung geschützt.

Bei Anwendung des vorgenannten Partikelstrahlverfahrens kann ein Schutz der Optik durch ein aufgebrachtes Gitter erfolgen, dessen Öffnungen kleiner sind als die verwendeten Partikel. Alternativ hierzu kann auch der äußere Teil der Fenster aus einem Material bestehen, welches transparent und härter als das verwendete abrasive Material ist. Bei Verwendung einer transparenten elastischen Folie kann vorteilhafterweise diese so gestaltet sein, daß zumindest der vordere Teil (Behandlungskopf), der die optischen Teile enthält abgedeckt wird; denkbar und im Rahmen der Erfindung liegt es auch, das gesamte Bearbeitungshandstück mit einer solchen Hülle zu überziehen, die nach Gebrauch abgezogen und desinfiziert bzw., bei einem Einmalartikel, entsprechend entsorgt wird.

Mehrere Ausführungsbeispiele der Erfindung werden nachfolgend anhand der Zeichnungen näher erläutert.
Es zeigen:
Figur 1 eine erste Variante einer Abschirmung mit flexiblem Schlauch zur Erläuterung des erfindungsgemäßen Verfahrens,
Figur 2 eine zweite Variante einer Abschirmung mit Spanngummi mit Darstellung einer Behandlung von vorn mit einem Kombinationshandstück,
Figur 3 eine weitere Alternative einer Abschirmung, dargestellt am Beispiel einer indirekten Behandlung von hinten, wobei Bearbeitungshandstück und Videokamera baulich voneinander getrennt sind,
Figuren 4 und 5 zwei Versionen eines Bearbeitungshandstückes mit integrierter Video-Kamera,
Figur 6 und 7 jeweils eine Video Kamera mit Saugkanüle kombiniert.

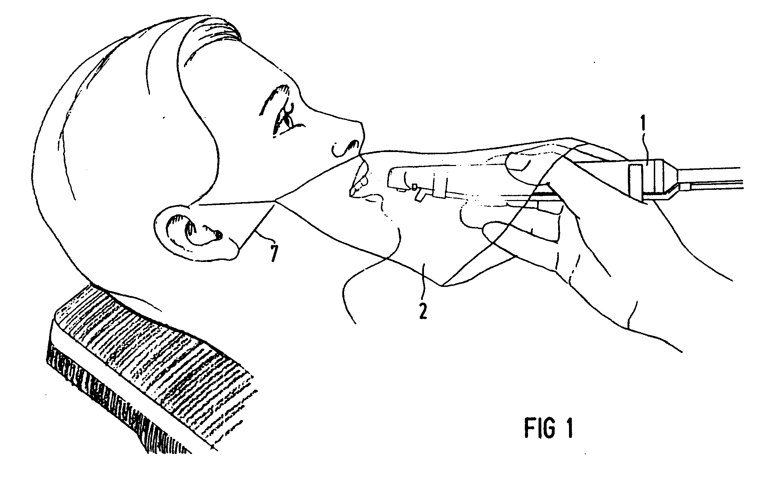

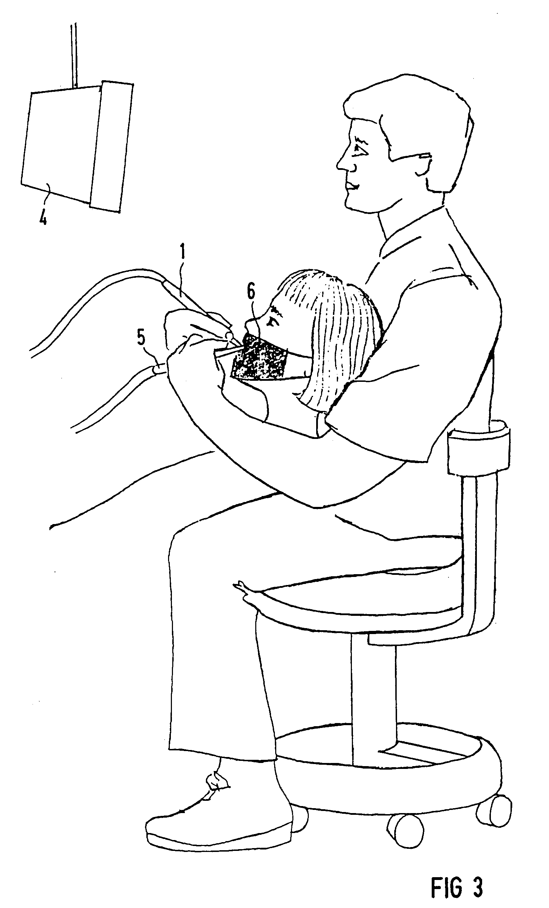

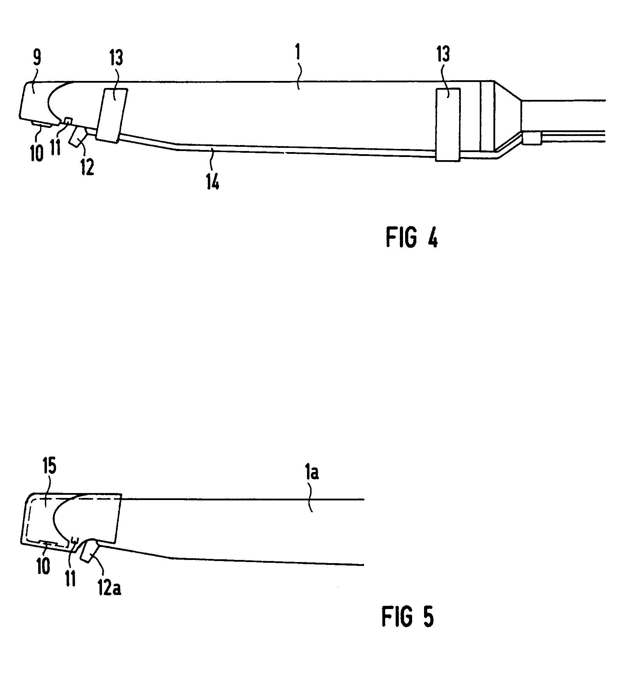

Anhand der Figur 1 wird die erfindungsgemäße Vorrichtung näher erläutert. Mit einem in Fig. 4 dargestellten und später noch näher erläuterten Bearbeitungshandstück 1, welches Mittel zur Bearbeitung von Zahnhartsubstanz sowie Mittel zur Beleuchtung des Präparationsfeldes und eine intraorale Videokamera enthält, deren Bild auf einem in Fig. 3 dargestellten Monitor wiedergebbar ist, kann in indirekter Behandlungstechnik wie folgt gearbeitet werden:

Um den Mund des Patienten wird die eine Öffnung eines flexiblen Schlauches 2 gelegt (gespannt, geklebt) und mit geeigneten Befestigungsmitteln 7 am Patientenmund fixiert.

Zur Behandlung greift der Behandler mit dem Bearbeitungshandstück durch die andere Öffnung des Schlauches in den Patientenmund. Das Handstück enthält in diesem Falle ein Abtragungswerkzeug, eine Video-Kamera und eine Beleuchtungseinrichtung.

In indirekter Behandlungstechnik, also mit Blickkontakt zu einem in Fig. 3 dargestellten Monitor 4, kann die Präparationsstelle bearbeitet werden. Die dabei durch zugeführte Kühlmittel oder auch durch die Bearbeitungsmittel (Salz/Sand) entstehende Aerosolwolke wird mit einer in Fig. 3 dargestellten üblichen Absaugkanüle 5, die auf gleiche Weise in den Patientenmund einführbar ist, abgesaugt. Durch den Schlauch 2, der auch transparent sein kann, ist optimaler Schutz gegen das Austreten von Keimen aus dem Patientenmund gegeben. Bei Anwendung eines Salz- oder Sandstrahlverfahrens können die anfallenden abrasiven Teilchen auf die gleiche Weise aufgefangen werden.

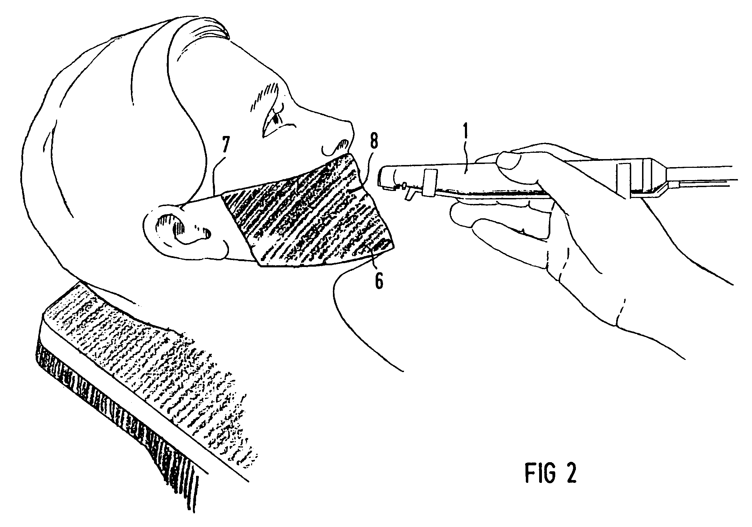

Die Figur 2 zeigt eine Alternative. Bei dieser ist über den Patientenmund eine vorzugsweise elastische Binde 6 (ähnlich einem Kofferdamm) gespannt, welche durch geeignete Befestigungsmittel 7 am Patientenkopf fixiert wird. Im Bereich der Mundöffnung enthält die Binde 6 einen oder mehrere Schlitze 8, über die das Bearbeitungshandstück 1 eingeführt werden kann.

Fig. 3 zeigt eine Behandlung in 12-Uhr-Position mit einem Monitor 4, der etwa in 6-Uhr-Position ausgerichtet ist.

Das Bearbeitungshandstück 1 ist auf herkömmliche Weise eigenständig mit Kühlmedium und Licht ausgestattet. Es kann ein rotierendes Instrument, ein Laser-, Sandstrahl- oder sonstiges Instrument sein.

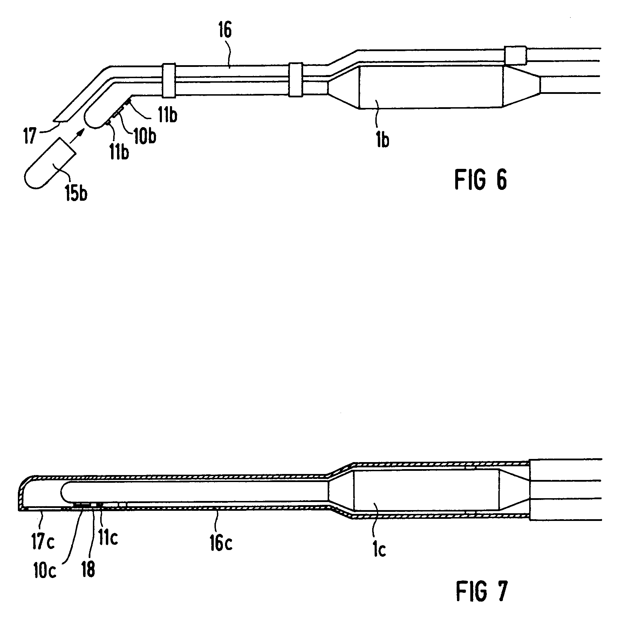

Die Video-Kamera ist bei diesem Ausführungsbeispiel, wie in Fig. 6 und 7 gezeigt, mit der Saugkanüle 5 kombiniert und wird durch Arzt (wie dargestellt) oder die Helferin getrennt eingeführt. Beide Handstücke werden durch die Abschirmfolie 6 gesteckt.

Die Figur 4 zeigt das nach Art eines Winkelstückes ausgebildete Bearbeitungshandstück in einer Seitenansicht. In einem mit 9 bezeichneten Kopfgehäuse ist die Aufnahmeoptik für eine im Innern des Handstückes angeordnete Intraoral-Videokamera untergebracht. Der Aufbau eines solchen Bearbeitungshandstückes mit Video-Kamera ist beispielsweise aus US-5 049 070 oder WO 91/03209 bekannt, so daß davon abgesehen wird, diesen Aufbau im einzelnen aufzuzeigen. Ein stirnseitig des Kopfgehäuses 9 vorgesehenes optisches Fenster für den Lichtaus- und -eintritt ist mit 10 angedeutet. Mit 11 ist das Lichtaustrittsende einer zusätzlich vorhandenen Beleuchtungseinrichtung bezeichnet, mit der die Präparationsstelle ausgeleuchtet werden kann. Mit 12 ist die Austrittsdüse eines am Bearbeitungshandstück 1 mittels geeigneter Haltemittel befestigbaren Zuleitungsrohres 14 bezeichnet, mit dem abrasive Partikel mit hoher Energie unter Zufuhr von Luft und gegebenenfalls Wasser als Bindemittel zuführbar sind.

Die Figur 5 zeigt eine Ausführungsform, bei der das Zuleitungsrohr 14 im Griffkörper des Bearbeitungshandstükkes integriert angeordnet ist. Das optische Fenster 10 sowie das Austrittsfenster 11 der Beleuchtungseinrichtung sind hier mittels eines auf das Kopfgehäuse aufsetzbaren Schutzhülle 15 geschützt. Die Hülle ist zumindest im Bereich der Austrittsfenster der Optik transparent und schützt deren Oberflächen vor Beschädigung, insbesondere durch die an der Austrittsdüse 12 austretenden abrasiven Teilchen.

Fig. 6 zeigt ein Intraoralkamera-Handstück mit einem Objektiv 10, einer Beleuchtung 11 und einer aufgeklipsten Absaugkanüle 16. Die optischen Teile sind durch einen in Pfeilrichtung aufschiebbaren durchsichtigen Überzug 15 zu schützen.

Fig. 7 zeigt ein Ausführungsbeispiel, bei dem die Absaugkanüle 16 über das Intraoralkamera-Handstück 1 gesteckt wird. Die Kanüle kann Fenster 18 zum Schutze der optischen Teile der Kamera enthalten.

Die Öffnung der Absaugkanüle ist mit 17 bezeichnet.

Um große Druckschwankungen im Munde zu vermeiden, ist es zweckmäßig, die Absaugleistung an den Spray- bzw. Sandstrahlstrom anzupassen. Dafür sind im Behandlungsplatz Mittel vorzusehen, die die Saugleistung so regeln, daß im Mund stets Druckausgleich oder ein geringer Unterdruck herrscht.

## Patentansprüche

1. Vorrichtung zur Bearbeitung von Zahnhartsubstanz in indirekter Behandlungstechnik, mit einem in den Patientenmund einführbaren Bearbeitungshandstück (1), mit dem ein Kühlmedium wie Luft und/oder Wasser der Präparationssstelle zugeführt werden kann, in Verbindung mit einer Absaugeinrichtung und einer intraoralen Videokamera, deren Zielobjekt auf einem Monitor wiedergegeben wird, **gekennzeichnet durch** eine am Patientenmund anzuordnende, zumindest den Patientenmund bedeckende und mit einer Öffnung versehene flexible Abschirmung (2), die so gestaltet ist, daß einerseits das Bearbeitungshandstück (1) in den Patientenmund ein- und zur Präparationsstelle hingeführt werden kann, und die andererseits durch ihre Flexibilität das eingeführte Bearbeitungshandstück (1) soweit umschließt, daß der Austritt von Partikeln aus dem Patientenmund verhindert wird.

2. Vorrichtung nach Anspruch 1,
**dadurch gekennzeichnet,**
daß als Abschirmung ein schlauchförmiges Gebilde (2) aus flexiblem, sich den Körperkonturen der Hand und des Bearbeitungshandstückes anpassendem Material vorgesehen ist.

3. Vorrichtung nach Anspruch 1,
**dadurch gekennzeichnet,**
daß als Abschirmung eine über den Patientenmund zu legende, vorzugsweise zu spannende Binde (6) vorgesehen ist.

4. Vorrichtung nach einem der Ansprüche 1 bis 3,
**dadurch gekennzeichnet,**
daß die Abschirmung als Einmalartikel ausgebildet ist.

5. Vorrichtung nach einem der Ansprüche 1 bis 4,
**dadurch gekennzeichnet,**
daß zur Bearbeitung von Zahnhartsubstanz dem Bearbeitungshandstück (1) außer Luft und gegebenenfalls Wasser ein abrasives Pulver zugeführt wird, welches dem Luftstrom beigegeben wird, wobei das mit Partikeln versetzte Gemisch aus einer am Bearbeitungshandstück (1) angeordneten und auf die Präparationsstelle gerichteten Düsenanordnung (12) mit hoher Geschwindigkeit austritt.

6. Vorrichtung nach Anspruch 5,
**dadurch gekennzeichnet,** daß die
Düsenanordnung (12) und ein mit ihr verbundenes Zuleitungsrohr (14) lösbar am Bearbeitungshandstück (1) befestigt ist.

7. Vorrichtung nach Anspruch 5,
**dadurch gekennzeichnet,**
daß die Düsenanordnung (12) und ein mit ihr verbundenes Zuleitungsrohr (14) integraler Bestandteil des Bearbeitungshandstückes (1) sind.

8. Vorrichtung nach einem der Ansprüche 1 bis 7,
**dadurch gekennzeichnet,**
daß das Bearbeitungshandstück (1) mit der Absaugeinrichtung kombiniert ist.

9. Vorrichtung nach Anspruch 1,
**dadurch gekennzeichnet,**
daß die Kamera mit einer Absaugkanüle (16) kombiniert ist.

10. Vorrichtung nach Anspruch 9,
**dadurch gekennzeichnet,**
daß die Absaugkanüle (16) auf die Kamera aufgeklipst ist.

11. Vorrichtung nach Anspruch 9,
**dadurch gekennzeichnet,**
daß die Absaugkanüle (16) fest mit der Kamera verbunden ist.

12. Vorrichtung nach Anspruch 9,
**dadurch gekennzeichnet,**
daß die Absaugkanüle (16) als Hülse über die Kamera gesteckt wird, so daß sie gleichzeitig als Schutz gegen Verletzung der optischen Teile dient.

13. Vorrichtung nach einem der Ansprüche 1 bis 11,
**dadurch gekennzeichnet,**
daß das optische Fenster (10) der Videokamera und gegebenenfalls benachbart angeordnete Fenster (11) einer Beleuchtungsein richtung mit einem Überzug (15) versehen sind, welcher zumindest im Bereich der Fenster transparent ist.

14. Vorrichtung nach Anspruch 13,
**dadurch gekennzeichnet,**
daß der Überzug (15) leicht wechselbar, vorzugsweise als Einmalartikel, ausgebildet ist.

15. Vorrichtung nach 1,
**dadurch gekennzeichnet,**
daß Mittel vorgesehen sind, die die Absaugleitung an den zugeführten Luft-/Wasserstrom bzw. an den zugeführten Strom des abrasiven Mediums so anpassen, daß im Patientenmund ein Druckausgleich oder ein geringer Unterdruck vorhanden ist.

## Claims

1. Apparatus for treating hard dental substances in an indirect treatment technique, having a treatment handpiece (1) which can be inserted in the patient's mouth and by means of which a cooling medium such as air and/or water can be supplied to the treatment location, together with a suction device and an intraoral video camera whose target subject is reproduced on a monitor, **characterised by** a flexible shielding means (2) to be arranged at the patient's mouth covering at least the patient's mouth and being provided with an opening, which shielding means is formed so that on the one hand the treatment handpiece (1) can be inserted in the patient's mouth and brought to the preparation location and on the other hand, owing to its flexibility, the shielding means surrounds the inserted treatment handpiece (1) so that the emergence of particles from the patient's mouth is prevented.

2. Apparatus according to claim 1, **characterised in that** a tubular structure (2) of flexible material which conforms to the physical contours of the hand and of the treatment handpiece, is provided as the shielding means.

3. Apparatus according to claim 1, **characterised in that** a preferably tensible bandage (6) to be put over the patient's mouth, is provided as the shielding means.

4. Apparatus according to one of claims 1 to 3, **characterised in that** the shielding means is formed as a disposable article.

5. Apparatus according to one of claims 1 to 4, **characterised in that**, for treating hard dental substances, there is supplied to the treatment handpiece (1), in addition to air and possibly water, an abrasive powder which is added to the flow of air, wherein the mixture mixed with particles exits at high speed from a nozzle arrangement (12) which is arranged on the treatment handpiece (1) and which is directed towards the preparation location.

6. Apparatus according to claim 5, **characterised in that** the nozzle arrangement (12) and a supply tube (14) connected thereto are detachable secured to the treatment handpiece (1).

7. Apparatus according to claim 5, **characterised in that** the nozzle arrangement (12) and a supply tube (14) connected thereto form an integral component of the treatment handpiece (1).

8. Apparatus according to one of claims 1 to 7, **characterised in that** the treatment handpiece (1) is combined with the suction device.

9. Apparatus according to claim 1, **characterised in that** the camera is combined with a suction cannula (16).

10. Apparatus according to claim 9, **characterised in that** the suction cannula (16) is clipped on to the camera.

11. Apparatus according to claim 9, **characterised in that** the suction cannula (16) is rigidly connected to the camera.

12. Apparatus according to claim 9, **characterised in that** the suction cannula (16) is slipped over the camera as a sheath so that it serves simultaneously as protection against damage to the optical parts.

13. Apparatus according to one of claims 1 to 11, **characterised in that** the optical window (10) of the video camera and any windows (11) of an illumination device that are arranged adjacently, are provided with a cover (15) which is transparent at least in the region of the windows.

14. Apparatus according to claim 13, **characterised in that** the cover (15) is formed so that it can be easily changed, preferably being formed as a disposable article.

15. Apparatus according to claim 1, **characterised in that** there are provided means which adapt the suction line to the flow of air/water supplied or to the flow of abrasive medium supplied so that there is an equalisation of pressure or a slight underpressure in the patient's mouth.

## Revendications

1. Dispositif d'usinage de l'émail d'une dent, comportant une pièce (1) à main d'usinage qui peut être introduite dans la bouche du patient et par laquelle un fluide de refroidissement comme de l'air et/ou de l'eau peut être envoyé au point à traiter, en liaison avec un dispositif d'aspiration et une caméra vidéo intraorale, dont la cible est reproduite sur un moniteur, caractérisé par un dispositif (2) de protection souple qui est à monter sur la bouche du patient, qui recouvre au moins la bouche du patient, qui est muni d'une ouverture et qui est réalisé de manière à pouvoir d'une part introduire la pièce (1) à main d'usinage dans la bouche du patient et l'amener au point à traiter et qui, d'autre part, en raison de sa souplesse, enferme la pièce (1) à main d'usinage au point d'empêcher la sortie de particules de la bouche du patient.

2. Dispositif suivant la revendication 1, caractérisé en ce qu'il est prévu comme dispositif de protection un produit (2) en forme de manchon souple en un matériau souple s'adaptant au contours corporels, à la main et à la pièce à main d'usinage.

3. Dispositif suivant la revendication 1, caractérisé en ce qu'il est prévu comme dispositif de protection un bandeau (6) à placer sur la bouche du patient, de préférence à tendre.

4. Dispositif suivant l'une des revendications 1 à 3, caractérisé en ce que le dispositif de protection est réalisé en article jetable.

5. Dispositif suivant l'une des revendications 1 à 4, caractérisé en ce que, pour usiner de l'émail d'une dent, on envoie à la pièce (1) à main d'usinage, outre de l'air et éventuellement de l'eau, une poudre abrasive, qui est ajoutée au courant d'air, le mélange mélangé à des particules sortant à grande vitesse d'un dispositif (12) à buse monté sur la pièce (1) à main d'usinage et dirigé sur le point à traiter.

6. Dispositif suivant la revendication 5, caractérisé en ce que le dispositif (12) à buse avec un tuyau (14) d'alimentation qui y est relié est fixé de manière amovible à la pièce (1) à main d'usinage.

7. Dispositif suivant la revendication 5, caractérisé en ce que le dispositif (12) à buse et un tuyau (14) d'alimentation font partie intégrante de la pièce (1) à main d'usinage.

8. Dispositif suivant l'une des revendications 1 à 7, caractérisé en ce que la pièce (1) à main d'usinage est combinée au dispositif d'aspiration.

9. Dispositif suivant la revendication 1, caractérisé en ce que la caméra est combinée à une canule (16) d'aspiration.

10. Dispositif suivant la revendication 9, caractérisé en ce que la canule (16) d'aspiration est rattachée par pince à la caméra.

11. Dispositif suivant la revendication 9, caractérisé en ce que la canule (16) d'aspiration est reliée de manière fixe à la caméra.

12. Dispositif suivant la revendication 9, caractérisé en ce que la canule (16) d'aspiration est mise comme un manchon sur la caméra, si bien qu'elle sert en même temps de dispositif de protection empêchant que les pièces d'objectif soient endommagées.

13. Dispositif suivant l'une des revendications 1 à 11, caractérisé en ce que la fenêtre (10) d'objectif de la caméra vidéo et éventuellement des fenêtres d'un dispositif d'éclairage montées à proximité sont munies d'un revêtement (15) qui est transparent au moins dans la zone des fenêtres.

14. Dispositif suivant la revendication 13, caractérisé en ce que le revêtement (15) est réalisé de manière à pouvoir être remplacé facilement, de préférence en article jetable.

15. Dispositif suivant la revendication 1, caractérisé en ce qu'il est prévu des moyens qui adaptent le conduit d'aspiration au courant d'air et/ou d'eau envoyé et au courant du fluide abrasif envoyé, de sorte qu'il y a une compensation de pression dans la bouche du patient ou qu'il y règne une petite dépression.
